# EUROPÄISCHE PATENTANMELDUNG

(11) **EP 1 920 891 A1**
(43) Veröffentlichungstag der Anmeldung: **14.05.2008**
(21) Anmeldenummer: 07021786.4
(22) Anmeldetag: 09.11.2007
(51) Int. Cl.: B25H 1/20, B08B 15/02

(54) **Sicherheitswekbank mit in Abhängigkeit der Frontscheibenposition steuerbarer Gebläseleistung**

(30) Priorität: 10.11.2006 DE 102006053122
(71) Anmelder: Thermo Electron LED GmbH, 63505 Langenselbold (DE)
(72) Erfinder: Ross, Gerd, 60388 Frankfurt (DE); Stahl, Hermann, 61130 Nidderau-Ostheim (DE); Reinhardt, Heiko, 63755 Alzenau (DE)
(74) Vertreter: Tomerius, Isabel

(57) **Zusammenfassung**

Die Erfindung betrifft eine Sicherheitswerkbank (1) mit einem von einem Gehäuse (2) umgebenden Arbeitsraum (3), mit einer in der Gehäusefrontseite (4) befindlichen und mit einer verstellbaren Frontscheibe (5) einstellbaren Arbeitsöffnung (6) zum Einlassen eines in den Arbeitsraum (3) einströmenden Außenluftstroms (7) und mit wenigstens einem Gebläse (8) zum Fördern des Außenluftstroms (7) in die Sicherheitswerkbank (1). Zur Verbesserung der Arbeitsbedingungen der Sicherheitswerkbank (1) für Bedienpersonen bei gleichzeitiger Sicherstellung eines zu jeder Zeit ausreichenden Personenschutzes ist eine Regeleinrichtung (41) und/oder eine Steuereinrichtung (15) vorhanden, durch die die Förderleistung des wenigstens einen Gebläses (8) in Abhängigkeit der Frontscheibenposition regelbar und/oder steuerbar ist.

## Beschreibung

Die Erfindung betrifft eine Sicherheitswerkbank mit einem von einem Gehäuse umgebenen Arbeitsraum, mit einer in der Gehäusefrontseite befindlichen und mit einer verstellbaren Frontscheibe einstellbaren Arbeitsöffnung zum Einlassen eines in den Arbeitsraum einströmenden Außenluftstroms, und mit wenigstens einem Gebläse zur Förderung des Außenluftstroms in die Sicherheitswerkbank.

Derartige Sicherheitswerkbänke und insbesondere solche zur Bearbeitung mikrobiologischer Proben, wie sie beispielsweise in der DE 44 41 784 C2 und der DE 100 17 196 A1 beschrieben sind, schützen vor Kontamination durch Bioaerosole, die bei mikrobiologischen Arbeiten auftreten und freigesetzt werden. Innerhalb der Sicherheitswerkbänke werden die mit Bioaerosolen kontaminierten Luftströme mit Hilfe von Gebläsen als gerichtete Luftströme fortgeführt und über Filter geleitet, welche die Verunreinigung aus dem Luftstrom zurückhalten. Bei diesen Filtern handelt es sich um Hochleistungs-Schwebstofffilter, beispielsweise HOSCH- oder HEPA-Filter, welche in der Lage sind, die im Luftstrom vorhandenen Mikroorganismen herauszufiltern. Wenigstens ein Teil der so gefilterten Luft wird als Abluftstrom aus der Sicherheitswerkbank abgeführt und in die Umgebungsluft der Sicherheitswerkbank eingeleitet.

Sicherheitswerkbänke unterscheiden sich in ihren Sicherheitsvorkehrungen und werden entsprechend den unterschiedlichen internationalen Normen gebaut, geprüft und zugelassen. In Abhängigkeit von ihrer Funktionsweise sind Sicherheitswerkbänke in die Klassen I, II und III unterteilt, wobei Sicherheitswerkbänke der Klasse I Personenschutz und Sicherheitswerkbänke der Klassen II und III zusätzlich zum Personenschutz auch Produktschutz bieten.

Bei Sicherheitswerkbänken der Klassen I und II wird der Personenschutz dadurch erreicht, dass Außenluft durch die Arbeitsöffnung ununterbrochen in den Arbeitsraum eingesaugt wird. Solange dieser Außenluftstrom nicht behindert ist und ausreichend Luft angesaugt wird, können Partikel und Aerosole nicht aus dem Innenraum der Sicherheitswerkbank nach außen gelangen. Die angesaugte Außenluft bildet also einen durch die Arbeitsöffnung strömenden Luftvorhang, der die an der Sicherheitswerkbank arbeitenden Personen bzw. die Umwelt vor Kontaminationen durch Partikel und Ähnliches schützt. Bei Sicherheitswerkbänken der Klasse III wird der Personenschutz durch Verschließen der Arbeitöffnung mit einem Gummi- oder Kunststoffvorhang sichergestellt, in dem sich handschuhähnliche, zum Arbeitsraum hin geschlossene Greiföffnungen befinden. Die vorliegende Erfindung betrifft Sicherheitswerkbänke der Klassen I und II.

Der von Sicherheitswerkbänken der Klassen II und III zusätzlich gewährte Schutz für Arbeitsgegenstände in der Werkbank vor Kontaminationen von außen oder vor Kontamination durch andere in der Werkbank befindliche Proben (Kreuzkontamination) wird dadurch erreicht, dass ein Teil des in die Werkbank eingesaugten Luftstroms nach dem Filtern wieder dem Innenraum als Umluftstrom zugeführt wird. Der Umluftstrom ist beim Produktschutz so ausgerichtet, dass er die in der Werkbank befindlichen Gegenstände umspült und somit verhindert, dass verunreinigte Luft mit diesen Gegenständen in Kontakt kommt.

Ein ausreichender Personenschutz ist Voraussetzung für den Betrieb von Sicherheitswerkbänken. Diese auch als Rückhaltevermögen bezeichnete Eigenschaft einer Sicherheitswerkbank wird zum Beispiel durch die Lufteintrittsgeschwindigkeit des Außenluftstroms im Bereich der Arbeitsöffnung definiert. In der DIN EN 12469 sind beispielsweise Mindestgrenzwerte für die Lufteintrittsgeschwindigkeit vorgeschrieben, die eingehalten werden müssen, um einen ausreichenden Personenschutz zu jedem Zeitpunkt zu gewährleisten.

Die Frontscheiben sind im Allgemeinen parallel zur Arbeitsöffnung höhenverstellbar ausgebildet. Dadurch ist die Höhe der Arbeitsöffnung variabel einstellbar, was wiederum einen direkten Einfluss auf die Lufteintrittsgeschwindigkeit des Außenluftstroms hat. Bei zunehmender Höhe der Arbeitsöffnung nimmt die Lufteintrittsgeschwindigkeit des Luftstroms proportional ab und umgekehrt. Daher ist es üblich, die Förderleistung der den Außenluftstrom fördernden Gebläse derart fest vorzugeben, dass auch bei maximaler Höhe der Arbeitsöffnung die Lufteintrittsgeschwindigkeit des Außenluftstromes einen Wert aufweist, der über dem vorgeschriebenen Minimalwert liegt. Dadurch ist ein Personenschutz jederzeit gewährleistet. Niedrigere Förderleistungen würden die Sicherheit der Bedienpersonen gefährden. Eine derartige Einstellung der Gebläse hat auf der anderen Seite zur Folge, dass die Schall- und Wärmeabstrahlungen der Sicherheitswerkbank relativ hoch sind. Zusätzlich kann es während des Betriebes zu Vibrationen kommen. Dies ist von einem ergonomischen Standpunkt aus betrachtet unerwünscht und verschlechtert die Arbeitsbedingungen von Bedienpersonen beim Arbeiten mit der Sicherheitswerkbank. Weiterhin können die auftretenden Vibrationen einen unerwünschten, negativen Einfluss auf die in der Sicherheitswerkbank befindlichen mikrobiologischen Proben haben.

Daher ist es **Aufgabe** der vorliegenden Erfindung, eine Sicherheitswerkbank anzugeben, bei der die Arbeitsbedingungen für Bedienpersonen verbessert sind und gleichzeitig ein zu jeder Zeit ausreichender Personenschutz sichergestellt ist.

Die Lösung dieser Aufgabe gelingt gemäß Anspruch 1 dadurch, dass eine Sicherheitswerkbank der eingangs genannten Art eine Regeleinrichtung und/oder eine Steuereinrichtung aufweist, durch die die Förderleistung des wenigstens einen Gebläses in Abhängigkeit von der Frontscheibenposition regelbar und/oder steuerbar ist. Bevorzugte Ausführungsformen sind den Unteransprüchen zu entnehmen.

Durch die Regel- bzw. Steuerbarkeit des wenigstens einen Gebläses ist es möglich, dessen Leistung an die Höhe der Arbeitsöffnung situationsbedingt anzupassen. Wird beispielsweise die Frontscheibe während des Betriebes von einer bestimmten Position aus hochgestellt, das heißt die Höhe der Arbeitsöffnung wird vergrößert, steuert die Steuereinrichtung das wenigstens eine Gebläse in der Weise an, dass dessen Förderleistung erhöht wird. Umgekehrt wird bei einer Verringerung der Arbeitsöffnungshöhe die Gebläseleistung heruntergefahren. Bei Verwendung einer Regeleinrichtung wird die Förderleistung so geregelt, dass die Lufteintrittsgeschwindigkeit, auch bei sich ändernder Arbeitsöffnungshöhe, ständig einem vorgegebenen Sollwert bzw. Sollwertebereich entspricht. Durch eine Erhöhung/Verringerung der Gebläseleistung kann die Verringerung/Erhöhung der Lufteintrittsgeschwindigkeit des Außenluftstroms, hervorgerufen durch eine vergrößerte/verkleinerte Arbeitsöffnung, kompensiert werden, so dass durchgängig ein ausreichender Personenschutz sichergestellt ist. Grundsätzlich können bei der erfindungsgemäßen Sicherheitswerkbank jeweils nur die Regel- oder die Steuereinrichtung oder beide Einrichtungen nebeneinander verwendet werden. Es ist auch eine integrierte Ausbildung der Regel- und der Steuereinrichtung möglich.

Vorteilhaft ist bei der vorliegenden Erfindung, dass die Förderleistung des wenigstens einen Gebläses an die Größe der Arbeitsöffnung in der Weise anpassbar ist, dass nur die jeweils für einen ausreichenden Personenschutz benötigte Förderleistung erzielt wird. Ist zum Beispiel ein Arbeitsgegenstand in die Sicherheitswerkbank einzuführen, wird die Arbeitsöffnung im Allgemeinen relativ weit geöffnet, so dass eine Bedienperson den Gegenstand unbehindert in die Sicherheitswerkbank einbringen kann. Dementsprechend regelt bzw. steuert die Regel- bzw. die Steuereinrichtung das wenigstens eine Gebläse so an, dass eine relativ hohe Förderleistung erzielt wird. Hat nun nach der Einbringung des Arbeitsgegenstandes in die Sicherheitswerkbank eine Bedienperson Arbeiten an diesem Gegenstand durchzuführen, ist die relativ große Arbeitsöffnungshöhe nicht mehr erforderlich und die Frontscheibe kann auf eine niedrigere Position abgesenkt werden, wodurch sich die Arbeitsöffnung verkleinert. Entsprechend wird die Förderleistung des Gebläses von der Regeleinrichtung bzw. der Steuereinrichtung heruntergefahren. Dadurch reduzieren sich für die Bedienperson während des Arbeitens die Schall- und Wärmeemissionen der Sicherheitswerkbank, und die Gefahr eines eventuellen Vibrierens der Sicherheitswerkbank bzw. des darin befindlichen Arbeitsgegenstandes wird verringert. Durch die vorliegende Erfindung werden also die Arbeitsbedingungen für die die Sicherheitswerkbank bedienende Person, unter gleichzeitiger Sicherstellung des Personenschutzes, verbessert.

Grundsätzlich kann die erfindungsgemäße Sicherheitswerkbank ein oder mehrere Gebläse aufweisen. Hierbei ist es bevorzugt, dass zumindest diejenigen Gebläse von der Regeleinrichtung und/oder der Steuereinrichtung regelbar und/oder steuerbar sind, die Einfluss auf den Außenluftstrom haben, also Außenluft durch die Arbeitsöffnung in den Arbeitsraum einsaugen. Die Regel- bzw. Steuerbarkeit der Förderleistung des wenigstens einen Gebläses wird im Allgemeinen dadurch erreicht, dass die Regeleinrichtung und/oder die Steuereinrichtung die Versorgungsspannung des wenigstens einen Gebläses reguliert, wodurch die Förderleistung an die jeweilige Frontscheibenposition anpassbar ist. Das wenigstens eine Gebläse kann sowohl ein Wechsel- als auch ein Gleichstromgebläse sein.

Zweckmäßig regelt bzw. steuert die Regeleinrichtung bzw. die Steuereinrichtung das wenigstens einen Gebläse in der Weise, dass sich die Lufteintrittsgeschwindigkeit des Außenluftstromes, unabhängig von der Frontscheibenposition, immer auf einen Wert einstellt, der im Bereich von ± 5%, besonders bevorzugt ± 2%, eines vorherbestimmten Wertes für die Lufteintrittsgeschwindigkeit liegt. Dadurch stellt sich näherungsweise ein konstanter Wert für die Lufteintrittsgeschwindigkeit ein. Der vorherbestimmte Wert für die Lufteintrittsgeschwindigkeit ist dabei so zu wählen, dass der Minimalwert des daraus resultierenden Wertebereiches gleich oder größer als der von den Normen für Sicherheitswerkbänke geforderte Mindestwert für die Lufteintrittsgeschwindigkeit ist. Die Vorgabe eines solchen Wertekorridors, in dem die Lufteintrittsgeschwindigkeit des Außenluftstromes liegen kann, ist zweckmäßig, da es zu zeitlichen Verzögerungen der Anpassung der Gebläseleistung bei Veränderungen der Frontscheibenposition kommen kann, so dass kleine Abweichungen vom vorgegebenen Wert für die Lufteintrittsgeschwindigkeit möglich sind. Weiterhin kann bei nur geringfügigen Änderungen der Fronscheibenposition unter Umständen von einer Änderung der Förderleistung abgesehen werden, wenn der neu einzustellende Wert für die Lufteintrittsgeschwindigkeit bei gleicher Gebläseleistung immer noch im zulässigen Wertekorridor liegt. Gewisse definierte Abweichungen und Schwankungen der Förderleistung des wenigstens einen Gebläses sind also in der erfindungsgemäßen Sicherheitswerkbank möglich. Bevorzugt sind diese Abweichungen jedoch möglichst gering zu halten, um einen optimalen Betrieb der Sicherheitswerkbank zu gewährleisten.

Um sicherzustellen, dass die Lufteintrittsgeschwindigkeit konstant bzw. innerhalb eines vorgegebenen Wertebereichs gehalten wird, ist es zweckmäßig, dass die Sicherheitswerkbank einen Strömungssensor zur Messung der Lufteintrittsgeschwindigkeit des Außenluftstroms im Bereich der Arbeitsöffnung aufweist. Bevorzugt wird der Strömungssensor in Strömungsrichtung hinter der Arbeitsöffnung angeordnet. Weiterhin ist es zweckmäßig, einen weiteren Strömungssensor im Bereich des Abluftstroms, der aus der Sicherheitswerkbank geführt wird, anzuordnen. Vorteilhafterweise ist der Strömungssensor in Strömungsrichtung hinter dem Abluftfilter angeordnet. Ist beispielsweise der Durchsatz eines Filters durch Verschmutzung reduziert, kann dies durch Messung des Strömungswertes des Abluftstroms festgestellt werden und die Gebläseleistung kann entsprechend angepasst werden. Hierdurch wird die Sicherheit der Sicherheitswerkbank weiter erhöht. Die Anordnung der Strömungssensoren zur Ermittlung des Strömungswertes der Lufteintritts- sowie der Abluftgeschwindigkeit ist nicht besonders beschränkt, sollte aber so erfolgen, dass störende und das Messergebnis verfälschende Einflüsse möglichst vermieden werden. Die Messergebnisse der Strömungssensoren werden bevorzugt in vorgegebenen Zeitintervallen an die Regel- und/oder die Steuereinrichtung weitergegeben und dort ausgewertet.

Um die Förderleistung des Gebläses so einstellen zu können, dass bei jeder beliebigen Frontscheibenposition gewährleistet ist, dass die Lufteintrittsgeschwindigkeit des Außenluftstroms konstant ist bzw. innerhalb eines vorgegebenen Wertebereichs liegt und sich somit ständig ein ausreichender Personenschutz einstellt, ist es zweckmäßig, einen Lufteintrittsgeschwindigkeitssollwert in der Regeleinrichtung zu speichern. Grundsätzlich kann der Sollwert auch ein Wertebereich sein. Die Regeleinrichtung wertet die vom Strömungssensor gemessenen Daten für die Lufteintrittsgeschwindigkeit aus und regelt die Förderleistung des Gebläses in der Weise, dass die gemessenen Werte der Lufteintrittsgeschwindigkeit im Wesentlichen dem Sollwert entsprechen. Wie bereits oben erwähnt, stellt sich der Sollwert für den Ist-Wert teilweise nur näherungsweise ein, und es können die o.a. Wertebereiche vorgegeben werden, in denen der Ist-Wert liegen muss. Zur Erhöhung der Sicherheit der Sicherheitswerkbank kann die Regeleinrichtung ausgebildet sein, weitere Parameter in die Regelung des Gebläses einzubeziehen. Bevorzugt ist zusätzlich zum Lufteintrittsgeschwindigkeitssollwert auch ein Abluftgeschwindigkeitssollwert in der Regeleinrichtung gespeichert. Hierdurch kann, beispielsweise bei einer Verstopfung der Filter, die Förderleistung des Gebläses auf einen Wert geregelt werden, bei dem eine ausreichende Abluftmenge aus der Sicherheitswertbank abgeführt werden kann. Die Regelung selbst wird analog zur Regelung der Lufteintrittsgeschwindigkeit durchgeführt. Grundsätzlich können noch weitere Parameter in der Regelung berücksichtigt werden. Hierdurch können neben der Arbeitsöffnungshöhe weitere Einflussgrößen auf die Lufteintrittsgeschwindigkeit (z.B. Verschmutzungsgrad der Filter, Luftwiderstände in der Abluftleitung) mit erfasst und ausgeregelt werden.

Vorteilhafterweise weist die Sicherheitswerkbank einen Positionssensor zur Messung der Frontscheibenposition auf. Dieser Positionssensor kann zum Beispiel unmittelbar an der Unterkante der Frontscheibe angeordnet sein und über elektrische Kontakte die Position der Frontscheibenkanten in ihren Führungsmitteln darstellen. Verwendbar sind aber auch andere geeignete Messeinrichtungen. Derartige Messeinrichtungen sind im Stand der Technik grundsätzlich bekannt und müssen hier nicht näher erläutert werden. Durch Vorhandensein eines derartigen Positionssensors ist die exakte Position der Frontscheibe bestimmbar, und die Steuereinrichtung kann das wenigstens eine Gebläse optimal steuern. Grundsätzlich kann die Positionsermittlung der Frontscheibe fortwährend oder diskret, das heißt intervallweise, erfolgen, wobei eine kontinuierliche Messung aufgrund der höheren Präzision bevorzugt ist. Alternativ zur Messung der Frontscheibenposition kann auch die Höhe der Arbeitsöffnung mittels eines entsprechenden Sensors gemessenen werden. Zweckmäßig sind in der Steuereinrichtung Werte für die Arbeitsöffnungsflächen in Abhängigkeit der Frontscheibenposition gespeichert, so dass die Steuereinrichtung einer jeweiligen Frontscheibenposition eine bestimmte Arbeitsöffnungsfläche zuordnen kann. Somit ist die Arbeitsöffnungsfläche, welche Einfluss auf die Lufteintrittsgeschwindigkeit hat, jederzeit bestimmbar.

Um die Förderleistung des wenigstens einen Gebläses an die jeweilig gemessene Frontscheibenposition anpassen zu können, ist es zweckmäßig, dass in der Steuereinheit vorherbestimmte Förderleistungswerte für das wenigstens eine Gebläse gespeichert sind. Jeder Förderleistungswert ist jeweils einer Frontscheibenposition zugeordnet. Die Steuereinrichtung ordnet der gemessenen Frontscheibenposition den jeweilig korrespondierenden Förderleistungswert zu und steuert das wenigstens eine Gebläse in der Weise an, dass sich die Förderleistung des wenigstens einen Gebläses im Wesentlichen auf den zugeordneten Förderleistungswert einstellt. Hierbei ist es bevorzugt, dass die Steuereinrichtung die Förderleistung des Gebläses so steuert, dass sich der tatsächliche Förderleistungswert in einem Wertekorridor von beispielsweise ± 5%, besonders bevorzugt von ± 2%, vom vorherbestimmten Förderleistungswert einstellt. Die gespeicherten Förderleistungswerte sind zweckmäßig so ausgewählt, dass die Lufteintrittsgeschwindigkeit der Sicherheitswerkbank, unabhängig von der Frontscheibenposition, annäherungsweise konstant bleibt. Die Einstellung solcher Wertekorridore ist zweckmäßig, da es, beispielsweise durch eine zeitverzögerte Anpassung der Förderleistung, zu geringfügigen Abweichungen kommen kann. Die Wertekorridore sind so zu wählen, dass ein ausreichender Personenschutz jederzeit gegeben ist. Grundsätzlich können in der Steuereinrichtung beliebig viele Förderleistungswerte für beliebig viele Frontscheibenpositionen gespeichert sein. Auch die Abstände zwischen den einzelnen Frontscheibenpositionen sind beliebig auswählbar. Zweckmäßig sind zumindest die Förderleistungswerte für diejenigen Frontscheibenpositionen gespeichert, die in der Praxis von Bedienpersonen verwendet werden, bzw. tatsächlich an der Sicherheitswerkbank einstellbar sind.

Alternativ oder zusätzlich kann in der Steuereinheit eine Formel gespeichert sein, mittels derer von der Steuereinrichtung für jede Frontscheibenposition ein optimaler Förderleistungswert ermittelbar ist. Die Steuereinrichtung steuert das wenigstens eine Gebläse in der Weise, dass sich eine Förderleistung einstellt, die dem ermittelten Förderleistungswert entspricht. Auch hier ist es bevorzugt, die tatsächliche Förderleistung auf einen Wert im Bereich eines Wertekorridors von ± 5%, besonders bevorzugt ± 2%, um den ermittelten Förderleistungswert einzustellen. Vorteilhaft ist bei der Verwendung einer derartigen Formel, dass für jede mögliche Frontscheibenposition ein Förderleistungswert ermittelbar ist. Zweckmäßig umfasst die Formel die Parameter Lufteintrittsgeschwindigkeit, Arbeitsöffnungsfläche und Gebläseleistung, wobei die Lufteintrittsgeschwindigkeit konstant und die beiden anderen Größen variabel sind. Die Arbeitsöffnungsfläche wird mittels Messung der Frontscheibenposition ermittelt, so dass dann die für das Konstanthalten der Lufteintrittsgeschwindigkeit notwendige Gebläseleistung berechenbar ist. Zu weiteren Verbesserung der Sicherheit der Sicherheitswerkbank ist es zweckmäßig, neben dem Steuern der Förderleistung in Abhängigkeit der Arbeitsöffnungshöhe die Förderleistung anhand weiterer Einflussgrößen, beispielsweise der vorhandenen Abluftgeschwindigkeit, zu regeln. Hierdurch können sämtliche auf die Lufteintrittsgeschwindigkeit wirkenden Einflussgrößen berücksichtigt und ausgeregelt werden.

Zur Speicherung der vorherbestimmten Sollwerte, Förderleistungswerte und/oder einer Formel ist es zweckmäßig, dass die Regeleinrichtung und/oder die Steuereinrichtung mindestens ein Speichermedium aufweist. Weiterhin ist es zweckmäßig, dass die Regeleinrichtung und/oder die Steuereinrichtung einen Prozessor umfasst, mittels dessen die notwendigen Rechenoperationen zum Vergleich der Soll- und Ist-Werte, zum Vergleich der übermittelten Messwerte mit den gespeicherten Werten und/oder zur Berechnung der Förderleistungswerte ausführbar sind. Grundsätzlich ist es auch möglich, dass die Regel- und die Steuereinrichtung auf ein gemeinsames Speichermedium zugreifen.

Damit bei der ersten Inbetriebnahme der Sicherheitswerkbank bzw. bei Wartungsarbeiten die Sollwerte, Förderleistungswerte und/oder die Formel zur Ermittlung der Förderleistung in das Speichermedium eingegeben werden können, ist es zweckmäßig, dass die Sicherheitswerkbank ein Terminal und/oder eine Schnittstelle aufweist, durch die eine entsprechende Eingabe erfolgen kann. Über die Schnittstelle kann die Sicherheitswerkbank beispielsweise mit einem zentralen Verwaltungssystem verbunden werden, so dass die Eingabe der Werte räumlich getrennt von der Sicherheitswerkbank erfolgen kann. Dies ist besonders vorteilhaft, wenn mehrere Sicherheitsbänke gleichzeitig gewartet werden bzw. in Erstbetrieb genommen werden müssen.

Grundsätzlich ist es möglich, die Sicherheitswerkbank so auszubilden, dass die Frontscheibe manuell von einer Bedienperson verstellbar ist. Bevorzugt ist es allerdings, dass die Frontscheibe mit Hilfe geeigneter Mittel automatisiert bewegbar ist. Hierfür ist beispielsweise ein Elektromotor mit einer entsprechenden Vorrichtung zur Bewegung der Frontscheibe geeignet. Dabei ist es zweckmäßig, dass an der Sicherheitswerkbank Taster oder Schalter vorgesehen sind, mittels denen die Verstellung der Frontscheibe von einer Bedienperson vorgenommen werden kann. Die Mittel zur Verstellung der Frontscheibe werden über den Taster oder Schalter angesteuert. Auch ist es möglich vorzusehen, dass die Frontscheibe mittels einer Fernbedienung verstellbar ist. Grundsätzlich kann die Verstellung der Frontscheibe stufenlos erfolgen, so dass Bedienpersonen die Höhe der Arbeitsöffnung auf die vorliegenden Arbeitsbedingungen individuell anpassen können. Hierbei ist es zweckmäßig, dass bei Verwendung einer Steuereinrichtung diese die Förderleistungswerte für das wenigstens eine Gebläse mittels einer im Speichermedium hinterlegten Formel ermittelt, da mittels der Formel für jede beliebige Scheibenposition ein Wert berechenbar ist. Im Hinblick auf die einzuhaltenden Sicherheitsnormen und den erforderlichen Rechenaufwand ist es allerdings bevorzugt, dass die Frontscheibe stufenweise zwischen verschiedenen vorgegebenen Positionen verstellbar ist. Besonders bevorzugt ist die Frontscheibe zwischen einer ersten und einer zweiten Arbeitsposition verstellbar. Selbstverständlich kann die Frontscheibe auch zusätzlich zu den zwei Arbeitspositionen in eine Schließposition gebracht werden, in der die Arbeitsöffnung ganz geschlossen ist. Bei dieser Ausführungsform ist die Frontscheibe also nur auf einige bestimmte Positionen einstellbar, die so ausgewählt sind, dass zwar sämtliche durchzuführenden Arbeiten in der Werkbank ausführbar sind, gleichzeitig aber sichergestellt ist, dass sämtliche Betriebsparameter, die für einen sicheren Arbeitsablauf notwendig sind, eingehalten werden. Zweckmäßig wird bei dieser Ausführungsform bei Verwendung einer Steuereinrichtung die Förderleistung des Gebläses von der Steuereinrichtung über vorherbestimmte Förderleistungswerte, die den jeweiligen vorgegebenen Arbeitspositionen zugeordnet sind, bestimmt, da dadurch die benötigte Prozessorleistung verringert werden kann.

Die erste Arbeitsposition ist zweckmäßig so ausgewählt, dass die Höhe der Arbeitsöffnung zwar relativ niedrig ist, aber noch genug Bewegungsfreiheit für eine Bedienperson lässt, die ihre Arme durch die Arbeitsöffnung in die Werkbank einführt, um an dort befindlichen Arbeitsgegenständen Arbeiten vorzunehmen. Bevorzugt weist die Arbeitsöffnung in der ersten Arbeitsposition ein Höhe von 10 bis 20 cm, besonders bevorzugt 13 bis 17 cm, auf. Höhen in diesen Bereichen bieten Bedienpersonen ausreichende Bewegungsfreiheit. Gleichzeitig wird die Größe der Arbeitsöffnung auf das benötigte Minimum reduziert, wodurch die Förderleistung des Gebläses von der Steuereinrichtung und/oder der Regeleinrichtung heruntergefahren werden kann und somit die Schall- und Wärmeemissionen der Sicherheitswerkbank verringert werden. Bevorzugt ist die erste Arbeitsposition weiterhin so ausgewählt, dass der mittlere Schallpegel der Sicherheitswerkbank während des Betriebes in einem Bereich von 50 bis 55 dB, besonders bevorzugt 52 bis 53 dB, liegt. Dies führt zu verbesserten Arbeitsbedingungen für die Bedienpersonen.

Die zweite Arbeitsposition ist zweckmäßig so gewählt, dass ausreichend Platz vorhanden ist, Arbeitsgegenstände in die Sicherheitswerkbank ein- bzw. auszuführen. Bevorzugt weist die Arbeitsöffnung in der zweiten Arbeitsposition eine Höhe von 30 bis 40 cm, besonders bevorzugt 33 bis 37 cm, auf. Dadurch weist die Arbeitsöffnung ausreichend Platz auf, so dass annähernd alle normalerweise in Sicherheitswerkbänke eingebrachten Arbeitsgegenstände sicher und bequem vom Bedienpersonal in die Sicherheitswerkbank ein- bzw. aus dieser herausgebracht werden können. Nach Ein- bzw. Ausfuhr der Arbeitsgegenstände wird die Frontscheibe wieder in die erste Arbeitsposition gebracht, und die Arbeiten an den Arbeitsgegenständen können in für die Bedienpersonen verbesserten Arbeitsbedingungen ausgeführt werden.

In einer weiteren bevorzugten Ausführungsform der Erfindung ist die Sicherheitswerkbank so ausgebildet, dass die Frontscheibe in Abhängigkeit vom Betriebszustand der Sicherheitswerkbank nur zwischen bestimmten Positionen verfahrbar ist. In welchem Betriebszustand die Sicherheitswerkbank sich gerade befindet, kann mit an sich bekannten und üblicherweise in einer Sicherheitswerkbank vorhandenen Kontroll- und Rückkopplungsvorrichtungen festgestellt werden, die beispielsweise an die Steuereinrichtung melden, ob die Werkbank ein- oder ausgeschaltet ist, Licht, Lüftung und Steckdosen des Gerätes an- oder abgeschaltet sind, ein Störungszustand vorliegt oder nicht, usw. Zweckmäßig ist im Speichermedium der Steuereinrichtung und/oder der Regeleinrichtung hinterlegt, in welche Positionen die Frontscheibe in einem jeweiligen Betriebszustand verfahrbar sein soll. Vorteilhaft ist hierbei, dass sich durch diese Positionsvorgaben die Frontscheibe immer nur in den für den jeweilig vorherrschenden Betriebszustand optimalen Positionen befindet. Befindet sich die Werkbank beispielsweise in einem Störungszustand, und wird dies an die Steuer- und/oder die Regeleinrichtung gemeldet, kann vorgegeben sein, dass in diesem Betriebszustand die Frontscheibe nur in die Schließposition oder nur in eine relativ niedrige Arbeitsposition verfahrbar ist, da aufgrund der Störung nicht gewährleistet ist, dass ein ausreichender Personenschutz bei einer großen Arbeitsöffnungshöhe garantiert ist. Dadurch wird die Sicherheit der Werkbank weiter verbessert.

Zur weiteren Verbesserung der Sicherheit der erfindungsgemäßen Sicherheitswerkbank ist es zweckmäßig, dass die Sicherheitswerkbank einen optischen und/akustischen Alarm ausgibt, wenn die gemessene Höhe der Arbeitsöffnung einen vorher bestimmten Maximalwert überschreitet. Dies kann zum Beispiel vorteilhaft sein, wenn die maximale Förderleistung des wenigstens einen Gebläses unter Einhaltung des Personenschutzes nur eine maximale Höhe der Arbeitsöffnung zulässt und bei einer diesen Maximalwert überschreitenden Höhe der Personenschutz nicht mehr gewährleistet werden kann. Weiterhin kann bei Sicherheitswerkbänken der Klasse 11 eine zu große Höhe der Arbeitsöffnung die Aufrechterhaltung der Umluftzirkulation und somit den Produktschutz gefährden. Es können auch mehrere, unterschiedliche Maximalwerte in Abhängigkeit des jeweiligen Betriebszustandes der Sicherheitswerkbank vorgegeben werden.

Es ist ferner zweckmäßig, dass die Werkbank wenigstens eine Messvorrichtung aufweist, die den Stromverbrauch und/oder die Drehzahl des wenigstens einen Gebläses ermittelt. Die Messergebnisse der in der Sicherheitswerkbank vorhandenen Strömungssensoren sowie der Messvorrichtung werden in vorgegebenen Zeitintervallen an ein Sicherheitsüberwachungssystem weitergegeben und dort ausgewertet. Zu diesem Zweck sind in einem Speichermedium des Sicherheitsüberwachungssystems Wertebereiche gespeichert, die angeben, innerhalb welcher Grenzen die Lufteintrittsgeschwindigkeit des Außenluftstroms sowie der Stromverbrauch oder die Drehzahl des wenigstens einen Gebläses liegen sollen. Anstatt im Sicherheitsüberwachungssystem ein Speichermedium auszubilden, kann dieses auch ausgebildet sein, auf ein Speichermedium der Regel- oder der Steuereinrichtung zuzugreifen. Generell kann das Sicherheitsüberwachungssystem auch integriert in eine der beiden Einrichtungen ausgebildet sein. Stellt das Sicherheitsüberwachungssystem eine Abweichung der gemessenen Parameter von den vorgegebenen Bereichen fest, wird zweckmäßig ein akustischer und/oder ein optischer Alarm ausgegeben. Auch ist es möglich, dass das Sicherheitsüberwachungssystem, beispielsweise im Fall einer Störung, weitere geeignete Maßnahmen, wie das Schließen der Arbeitsöffnung, veranlasst. Die Steuereinrichtung und/oder die Regeleinrichtung und das Sicherheitsüberwachungssystem können integriert als ein Bauteil oder separat ausgebildet sein. Zweckmäßig stehen die Komponenten zwecks Datenaustauschs in gegenseitiger Kommunikationsverbindung.

Weiterhin ist es zweckmäßig, dass die Regeleinrichtung und/oder die Steuereinrichtung so programmiert ist, dass das wenigstens eine Gebläse nur innerhalb der gespeicherten Wertebereiche für den Stromverbrauch und/oder die Drehzahl betrieben werden kann. So kann wirkungsvoll verhindert werden, dass das wenigstens eine Gebläse, beispielsweise durch zu große Stromzufuhr oder zu hohe Drehzahl, beschädigt wird. Ist ein Betrieb unter den vorgegebenen Bedingungen nicht möglich, löst das Sicherheitsüberwachungssystem der Werkbank einen akustischen und/oder optischen Alarm aus. Zusätzlich kann, wie in allen anderen Alarmfällen auch, die Ursache für den ausgehenden Alarm auf einem Display oder in sonstiger geeigneter Weise angezeigt werden.

Zweckmäßig weist das Sicherheitsüberwachungssystem der Sicherheitswerkbank zusätzlich Einrichtungen zur Überwachung der Funktionsfähigkeit wesentlicher Bauteile der Sicherheitswerkbank auf. Zusätzlich zur Einhaltung vorgegebener Betriebsparameter wird also überwacht, ob bestimmte Bauteile der Sicherheitswerkbank überhaupt betriebsbereit sind. Bevorzugt wird wenigstens eines der Bauteile Positionssensor, Strömungssensor, Messvorrichtung, Gebläse und Stromversorgung der Sicherheitswerkbank von dem Sicherheitsüberwachungssystem überwacht. Um einen sicheren Betrieb der Werkbank zu gewährleisten, werden zweckmäßig mehrere oder alle dieser Bauteile regelmäßig auf ihre ungestörte Betriebsfähigkeit hin überwacht. Wird ein Ausfall oder ein Defekt auch nur eines der überwachten Bauteile bemerkt, wird ein optischer und/oder akustischer Alarm ausgegeben. Von Vorteil ist vor allem die Überwachung der Steuereinrichtung und/oder der Regeleinrichtung durch ein unabhängiges Sicherheitsüberwachungssystem, da so sichergestellt wird, dass die von der Regeleinrichtung und/oder Steuereinrichtung geregelten bzw. gesteuerten Förderleistungen des wenigstens einen Gebläses den Vorgaben entsprechen und ein ausreichender Personenschutz garantiert ist.

Die mehrfache Kontrolle der wesentlichen Komponenten und Betriebsparameter der Sicherheitswerkbank ermöglichen eine besonders sichere Einhaltung der Vorschriften nach DIN EN 12469. Die zur Überwachung eingesetzten Sensoren sind an sich bekannt und müssen daher nicht näher erläutert werden.

Die Erfindung wird nachfolgend anhand einer Zeichnung näher erläutert. Darin zeigen schematisch:
- Fig. 1: eine Sicherheitswerkbank mit einer Frontscheibe in einer ersten Arbeitsposition;
- Fig. 2: die Sicherheitswerkbank aus Figur 1 mit der Frontscheibe in einer zweiten Arbeitsposition;
- Fig. 3: ein Querschnitt der in Figur 1 gezeigten Sicherheitswerkbank;
- Fig. 4: ein Querschnitt der in Figur 2 gezeigten Sicherheitswerkbank;
- Fig. 5: ein Schaltbild einer Sicherheitswerkbank mit Steuereinrichtung und Sicherheitsüberwachungssystem und
- Fig. 6: ein Schaltbild einer Sicherheitswerkbank mit Regeleinrichtung und Sicherheitsüberwachungssystem.

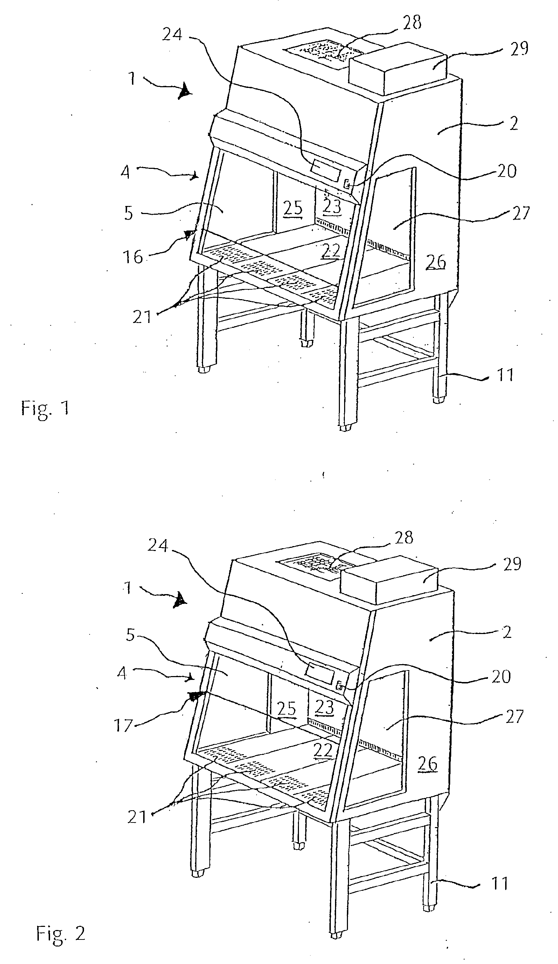

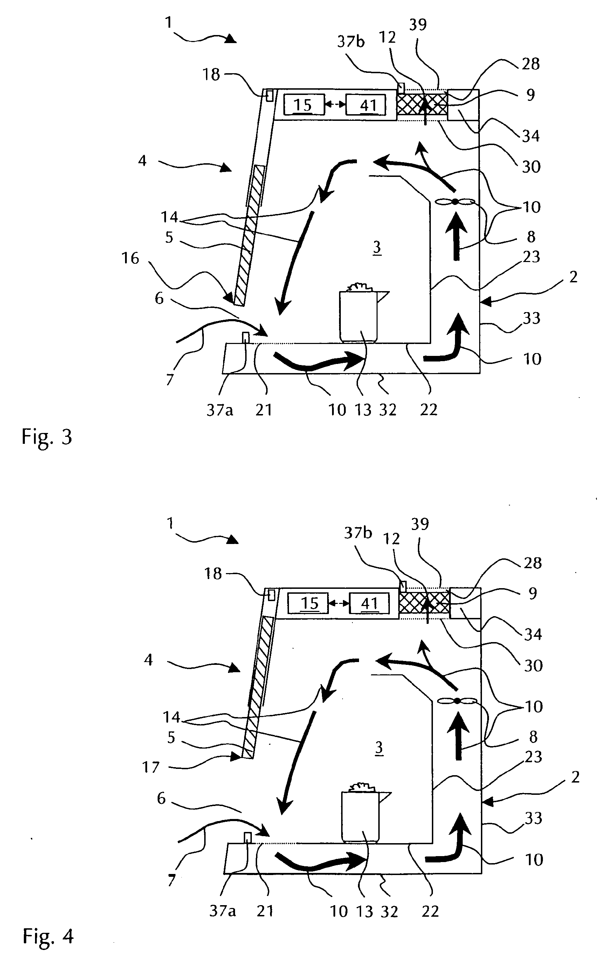

Die Figuren 1 bis 4 zeigen eine erfindungsgemäße Sicherheitswerkbank 1, die beispielsweise zur Bearbeitung mikrobiologischer Kulturen eingesetzt werden kann. In ihrem Grundaufbau entspricht die Sicherheitswerkbank 1 dem, was aus dem Stand der Technik bekannt ist. Die Sicherheitswerkbank 1 weist ein Gehäuse 2 auf, welches einen Arbeitsinnenraum 3 umgibt und auf einem Rahmengestell 11 aufsitzt. Der Arbeitsinnenraum 3 ist zur Aufnahme eines oder mehrerer Probenbehälter 13 ausgebildet, in denen sich mikrobiologische Kulturen befinden. An der Gehäusefrontseite 4 ist eine verstellbare Frontscheibe 5 angeordnet. Die Frontscheibe 5 ist so gelagert, dass sie im Wesentlichen parallel zur Gehäusefrontseite 4, zwischen einer ersten Arbeitsposition 16 (siehe Figuren 1 und 3) und einer zweiten Arbeitsposition 17 (siehe Figuren 2 und 4), hoch und herunter verschoben werden kann. Selbstverständlich kann die Frontscheibe 5 auch in eine den Arbeitsinnenraum 3 von der Umgebungsluft abschließende Schließposition gebracht werden.

Die Sicherheitswerkbank 1 ist so eingestellt, dass die Frontscheibe 5 zwischen insgesamt drei Positionen, der Schließposition, der ersten Arbeitsposition 16 und der zweiten Arbeitsposition 17, verstellbar ist. Die Höhe der Arbeitsöffnung 6 ergibt sich aus dem Spalt zwischen der Unterseite der Frontscheibe 5 und der Arbeitsraum-Bodenplatte 22 des Gehäuses 2. Die Höhe und somit auch die Fläche der Arbeitsöffnung 6 sind in der ersten Arbeitsposition 16 der Frontscheibe 5 kleiner als diejenigen der zweiten Arbeitsposition 17. Befindet sich die Frontscheibe 5 in der zweiten Arbeitsposition 17, ist die Höhe der Arbeitsöffnung 6 groß genug, dass eine Bedienperson einen Probenbehälter 13 in den Arbeitsinnenraum 3 ein- bzw. aus diesem herausführen kann. Befindet sich die Frontscheibe 5 dagegen in der ersten Arbeitsposition 16, ist die Höhe der Arbeitsöffnung 6 deutlich verringert, so dass der Probenbehälter 13 nicht mehr ein- bzw. ausgeführt werden kann. Allerdings verbleibt in der ersten Arbeitsposition 16 noch so viel Platz, dass eine Bedienperson in den Arbeitsraum 3 durch die Arbeitsöffnung 6 hineingreifen und Arbeiten an den in den Probenbehältern 13 befindlichen mikrobiologischen Kulturen durchführen kann. In der ersten Arbeitsposition 16 fährt die Steuereinrichtung 15 bzw. die Regeleinrichtung 41 die Förderleistung des Gebläses 8 gegenüber der zweiten Arbeitsposition 17 herunter, so dass sich in der ersten Arbeitsposition 16 ein reduzierter Schallpegel und eine geringere Wärmestrahlung der Sicherheitswerkbank 1 einstellen, wodurch die Arbeitsbedingungen für Bedienpersonen verbessert werden.

Das Verfahren der Frontscheibe 5 zwischen den einzelnen Positionen erfolgt durch Bedienung eines an der Gehäusefrontseite 4 angebrachten Wippentasters 20, der als Doppelwippe ausgeführt ist. Der Wippentaster 7 weist also eine obere und eine untere Wipptaste auf, wobei durch Betätigung der oberen Wipptaste die Frontscheibe 5 eine Position nach oben verfährt (z. B. von der ersten Arbeitsposition 16 in die zweite Arbeitsposition 17) und durch Betätigen der unteren Wipptaste entsprechend eine Position nach unten verfährt (z.B. von der ersten Arbeitsposition 16 in die Schließposition). Die Sicherheitswerkbank kann so eingestellt sein, dass durch das Betätigen des Wippentasters 20 neben dem Verfahren der Frontscheibe 5 auch andere Funktionen ausgelöst werden können. So kann der Wippentaster 20 beispielsweise so konfiguriert sein, dass durch das Betätigen der oberen Wipptaste zusätzlich zum Verfahren der Frontscheibe 5 von der Schließposition in die erste Arbeitsposition 16 auch die Stromversorgung der Sicherheitswerkbank 1 angeschaltet wird.

Neben dem Wippentaster 20 befindet sich an der Gehäusefrontseite 4 ein Display 24, mittels welchem dem Benutzer der Betriebszustand der Sicherheitswerkbank 1 und/oder Fehlermeldungen angezeigt werden.

Das Gehäuse 2 ist üblicherweise zweischalig aufgebaut, so dass das Gehäuse selbst zumindest teilweise als Lüftungskanal für die in der Sicherheitswerkbank zirkulierenden Luftströme dient. An den Seiten 25, 26 des Gehäuses befinden sich zwei Seitenscheiben 27. Die Seitenscheiben 27, wie auch die Frontscheibe 25, bestehen aus Glas, z.B. einem geeigneten Verbundglas. In der Arbeitsraum-Bodenplatte 22 sind im Bereich der Arbeitsöffnung 6 Ansaugöffnungen 21 angeordnet. Unterhalb und parallel zur Arbeitsraum-Bodenplatte 22 verläuft eine außenliegende Bodenplatte 32. Der Zwischenraum zwischen diesen beiden Platten 22, 32 bildet einen Luftführungskanal, durch den ein Luftstrom 10 von der Gehäusefrontseite 4 her in Richtung auf die Gehäuserückwand strömt. Im weiteren Strömungsverlauf wird der Luftstrom 10 durch den ebenfalls als Luftführungskanal ausgebildeten Zwischenraum zwischen innerer Rückwand 23 und äußerer Rückwand 33 nach oben geführt. Am oberen Ende dieses Lüftungskanals befindet sich das Gebläse 8, das den Luftstrom 10 ansaugt. Die Dicke der Strömungspfeile 7, 10, 12, 14 in den Figuren 2 und 4 verdeutlicht stark schematisiert die unterschiedlichen Volumina der jeweiligen Luftströme.

Ein Teil des vom Gebläse 8 angesaugten Luftstroms 10 strömt als Abluftstrom 12 durch den Abluftkanaleingang 30 in den Abluftkanal 28, der durch die obere Abdeckung 34 verlaufend ausgebildet ist, ein. Im Inneren des Abluftkanals 28 ist ein Filter 9 angeordnet, durch den der Abluftstrom 12 strömt. Der Filter 9 ist beispielsweise ein herkömmlicher HEPA-Filter, der Verunreinigungen wie Mikroorganismen aus der Umluft herausfiltert und so verhindert, dass mit dem Abluftstrom 12 Verunreinigung in die Außenluft getragen werden. Direkt hinter dem Filter 9 in Strömungsrichtung ist ein Strömungssensor 37b zur Messung der Abluftgeschwindigkeit angeordnet. Dieser Sensor 37b übermittelt die von ihm gemessenen Werte an die Regeleinrichtung 41, so das diese Einflussgröße ausgeregelt werden kann. Der Abluftstrom 12 kann im weiteren Verlauf durch den Abluftkanalausgang 39 nach außen strömen und der die Sicherheitswerkbank 1 umgebenden Außenluft oder einem hier nicht näher dargestellten gebäudeeigenen Abluftsystem zugeführt werden. Der größere Teil des Luftstroms 10 wird vom Gebläse 8 als Umluftstrom 14 ungefiltert zurück in den Arbeitsinnenraum 3 geleitet. Die aus der Sicherheitswerkbank 1 herausgeführte Abluft 12 wird durch einen durch die Arbeitsöffnung 6 in den Arbeitsinnenraum 3 hereinströmenden Außenluftstrom 7 ersetzt. Der Außenluftstrom 7 und der Abluftstrom 12 weisen in etwa die gleichen Strömungsmengen auf, so dass eine insgesamt konstante Luftströmungsmenge in der Sicherheitswerkbank 1 vorhanden ist. Der Außenluftstrom 7 wird vom Gebläse 8 angesaugt und vereinigt sich im Arbeitsinnenraum 3 mit dem Umluftstrom 14 zum Luftstrom 10. Die hier gezeigte Sicherheitswerkbank 1 bietet also ausschließlich Personenschutz und keinen Produktschutz und ist somit eine Sicherheitswerkbank der Klasse I.

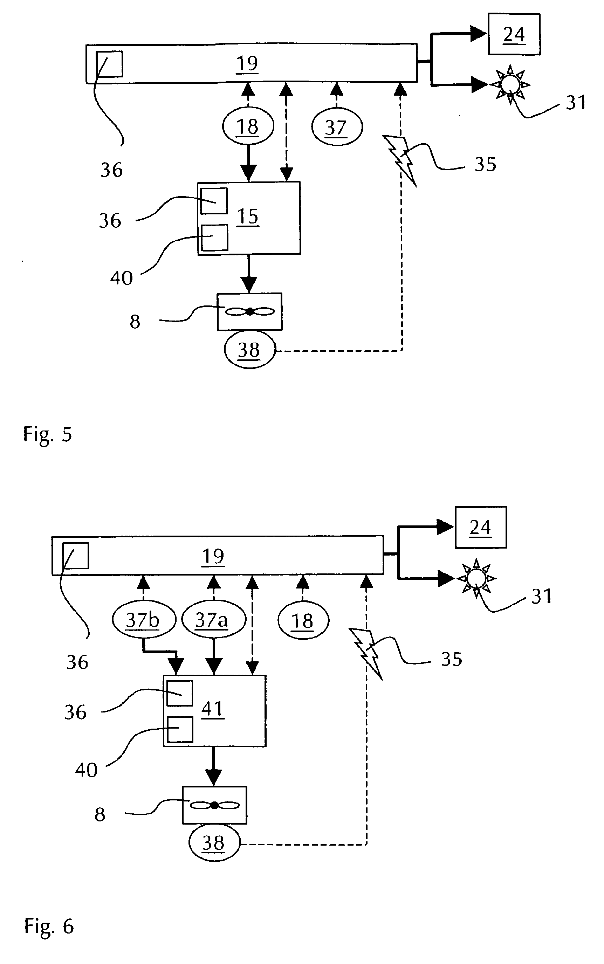

Um ständig eine optimal angepasste Leistung des Gebläses 8 erreichen zu können, wird die Position der Frontscheibe 5 in der Sicherheitswerkbank 1 überwacht. Aus diesem Grund ist im Bereich der Gehäusefrontseite 4 ein Positionssensor 18 angeordnet, der ständig die aktuelle Frontscheibenposition ermittelt und den Messwert an eine Steuereinrichtung 15 sendet (vgl. Figur 5). Die Steuereinrichtung 15 ist in der oberen Gehäuseabdeckung 34 angeordnet. Alternativ ist auch eine Anordnung im Schaltkasten 29 denkbar. In einem in der Steuereinrichtung 15 integriert ausgebildeten Speichermedium 36 wurden vor Inbetriebnahme der Sicherheitswerkbank 1 Förderleistungswerte des Gebläses 8 für jede mögliche Arbeitsposition gespeichert. Konkret sind im Speichermedium 36 der Steuereinrichtung 15 also ein Förderleistungswert für die erste Arbeitsposition 16 und ein weiterer Förderleistungswert für die zweite Arbeitsposition 17 gespeichert. Weiterhin weist die Steuereinrichtung einen Prozessor 40 auf, mittels dessen die notwendigen Rechenoperationen ausführbar sind. Die Steuereinrichtung 15 steuert die Stromversorgung des Gebläses 8 nun so, dass dieses einen Luftvolumenstrom 10 fördert, dessen Größe dem Förderleistungswert der jeweils vorhandenen Arbeitsposition entspricht. Die in dem Speichermedium 36 der Steuereinrichtung 15 gespeicherten Förderleistungswerte sind so gewählt, dass die Lufteintrittsgeschwindigkeit des Außenluftstroms 7 im Bereich der Arbeitsöffnung 6 auch bei unterschiedlichen Arbeitspositionen annähernd konstant ist. Die Lufteintrittsgeschwindigkeit ist weiterhin so eingestellt, dass ein Personenschutz jederzeit gewährleistet ist.

Um neben der Arbeitsöffnungshöhe weitere Einflussgrößen zu berücksichtigen, die auf die Lufteintrittsgeschwindigkeit Einfluss nehmen, ist neben der Steuereinrichtung 15 eine Regeleinrichtung 41 vorgesehen. Ebenso wie die Steuereinrichtung 15 ist die Regeleinrichtung 41 in der oberen Gehäuseabdeckung 34 angeordnet. Alternativ ist auch hier eine Anordnung im Schaltkasten 29 denkbar. Im vorliegenden Beispiel sind die Steuereinrichtung 15 und die Regeleinrichtung 41 getrennt voneinander ausgebildet, stehen aber in Kommunikation miteinander (angedeutet durch den gestrichelten Doppelpfeil). Grundsätzlich ist aber auch ein integrierte Bauweise denkbar. In einem in der Regeleinrichtung 41 integriert ausgebildeten Speichermedium 36 (vgl. Fig. 6) wurden vor Inbetriebnahme der Sicherheitswerkbank 1 ein Sollwert oder ein Sollwertbereich für die Abluftgeschwindigkeit gespeichert. Der Strömungssensor 37b übermittelt in kontinuierlichen Abständen die Ist-Werte der Abluftgeschwindigkeit an die Regeleinrichtung 41. Weicht der Ist-Wert, beispielsweise durch Filterverschmutzung, vom Soll-Wert ab, passt die Regeleinrichtung 41 die Förderleistung des Gebläses 8 in der Weise an, dass sowohl die Lufteintrittsgeschwindigkeit als auch die Abluftgeschwindigkeit immer in den vorgesehenen Wertebereichen liegen.

Um sicherzugehen, dass der Personenschutz nicht durch eine Betriebsstörung der Sicherheitswerkbank 1 gefährdet ist, ist im Bereich der Arbeitsöffnung 6 ein Strömungssensor 37a angeordnet, der die Lufteintrittsgeschwindigkeit misst und die gemessenen Werte an das Sicherheitsüberwachungssystem 19 liefert. Der Strömungssensor 37a ist beispielsweise ein Anemometer. Das Sicherheitsüberwachungssystem 19 kann beispielsweise im Schaltkasten 29 angeordnet sein. Bei der vorliegenden Ausführungsform ist das Sicherheitsüberwachungssystem 19 getrennt von der Steuereinrichtung 15 und der Regeleinrichtung 41 ausgebildet. Allerdings ist auch eine integrierte Bauweise denkbar. Diese Systeme stehen in Kommunikationsverbindung miteinander. Im Sicherheitsüberwachungssystem 19 ist ebenfalls ein Speichermedium 36 vorhanden, in welchem ein Sollwertebereich für die Lufteintrittsgeschwindigkeit hinterlegt ist. Liegt ein vom Strömungssensor 37a gemessener Wert der Lufteintrittsgeschwindigkeit außerhalb dieses Sollwertebereichs, veranlasst das Sicherheitsüberwachungssystem 19 eine Alarmvorrichtung 31, ein akustisches und/oder optisches Signal auszugeben, das den Benutzer darauf aufmerksam macht, dass eine Störung vorliegt. Weiterhin sendet das Sicherheitsüberwachungssystem 19 ein entsprechendes Signal an das Display 24, welches dem Benutzer den genauen Fehlerstatus anzeigt. Im Falle einer reinen Regelung (vgl. Fig. 6) übermittelt der Strömungssensor 37a zusätzlich die Ist-Werte der Lufteintrittsgeschwindigkeit an die Regeleinrichtung 41, die diese mit in ihrem Speichermedium 36 gespeicherten Sollwerten abgleicht und die Förderleistung des Gebläses 8 so regelt, dass sich der Ist-Wert dem Soll-Wert zumindest näherungsweise angleicht.

Zusätzlich zum Strömungssensor 37a überwacht das Sicherheitsüberwachungssystem 19 die Steuereinrichtung 15, die Regeleinrichtung 41, den Positionssensor 18 sowie eine Messvorrichtung 38 zur Messung des Stromverbrauchs und/oder der Drehzahl des Gebläses 8. In den Figuren 5 und 6 sind die zum Sicherheitsüberwachungssystem 19 gehörenden Rückmeldungen als gestrichelte Pfeile dargestellt. Funktioniert eine der Komponenten nicht ordnungsgemäß, veranlasst das Sicherheitsüberwachungssystem 19 ebenfalls die Ausgabe eines Alarmsignals durch die Alarmvorrichtung 31 bzw. die Ausgabe einer entsprechenden Meldung mittels des Displays 24. Darüber hinaus sind für den Positionssensor 18 sowie für die Messvorrichtung 38 Maximal- bzw. Minimalwerte in dem Speichermedium 36 des Sicherheitsüberwachungssystem 19 gespeichert, bei deren Über- bzw. Unterschreitung ebenfalls ein Alarm und eine entsprechende Meldung ausgegeben werden.

In den in den Figuren 5 und 6 gezeigten Schemata liegt beispielsweise eine Fehlfunktion des Gebläses 8 vor. Durch den Pfeil 35 schematisch angedeutet, wird diese Fehlermeldung von der Messvorrichtung 38 an das Sicherheitsüberwachungssystem 19 gemeldet, welches wiederum eine Alarmvorrichtung 31 sowie ein Display 34 ansteuert. Die Alarmvorrichtung 31 ist hier eine Lampe. Somit verdeutlicht ein optischer Alarm dem Benutzer der Sicherheitswerkbank 1, dass eine Fehlfunktion vorliegt. Weiterhin wird dem Benutzer über das Display 24 angezeigt, dass es sich um eine Fehlfunktion des Gebläses 8 handelt.

## Patentansprüche

1. Sicherheitswerkbank (1) mit einem von einem Gehäuse (2) umgebenen Arbeitsraum (3), mit einer in der Gehäusefrontseite (4) befindlichen und mit einer verstellbaren Frontscheibe (5) einstellbaren Arbeitsöffnung (6) zum Einlassen eines in den Arbeitsraum (3) einströmenden Außenluftstroms (7) und mit wenigstens einem Gebläse (8) zum Fördern des Außenluftstroms (7) in die Sicherheitswerkbank (1),
**dadurch gekennzeichnet,**
**dass** eine Regeleinrichtung (41) und/oder eine Steuereinrichtung (15) vorhanden ist, durch die die Förderleistung des wenigstens einen Gebläses (8) in Abhängigkeit von der Frontscheibenposition (16, 17) regelbar und/oder steuerbar ist.

2. Sicherheitswerkbank nach Anspruch 1,
**dadurch gekennzeichnet,**
**dass** die Regeleinrichtung (41) und/oder die Steuereinrichtung (15) ausgebildet ist, das wenigstens eine Gebläse (8) in der Weise zu regeln und/oder zu steuern, dass die Lufteintrittsgeschwindigkeit des Außenluftstroms (7) im Wesentlichen konstant ist.

3. Sicherheitswerkbank nach Anspruch 1 oder 2,
**dadurch gekennzeichnet,**
**dass** ein Strömungssensor (37) zur Messung der Lufteintrittsgeschwindigkeit des Außenluftstroms (7) im Bereich der Arbeitsöffnung (6) angeordnet ist.

4. Sicherheitswerkbank nach einem der vorhergehenden Ansprüche, bei der zumindest ein Teil des von dem wenigstens einen Gebläses (8) angesaugten Außenluftstroms (7) durch einen Abluftfilter als Abluftstrom aus der Sicherheitswerkbank (1) geblasen wird,
**dadurch gekennzeichnet,**
**dass** ein Strömungssensor (37) zur Messung der Geschwindigkeit des Abluftstroms (7) hinter dem Abluftfilter angeordnet ist.

5. Sicherheitswerkbank nach Anspruch 3 oder 4,
**dadurch gekennzeichnet,**
**dass** in der Regeleinrichtung (41) ein Lufteintrittsgeschwindigkeits-Sollwert gespeichert ist und die Regeleinrichtung (41) ausgebildet ist, die Förderleistung des wenigstens einen Gebläses (8) so zu regeln, dass die gemessenen Lufteintrittsgeschwindigkeitswerte im Wesentlichen dem Lufteintrittsgeschwindigkeits-Sollwert entsprechen.

6. Sicherheitswerkbank nach Anspruch 4 oder 5,
**dadurch gekennzeichnet,**
**dass** in der Regeleinrichtung (41) ein Abluftgeschwindigkeits-Sollwert gespeichert ist und die Regeleinrichtung (41) ausgebildet ist, die Förderleistung des wenigstens einen Gebläses (8) so zu regeln, dass die gemessenen Abluftgeschwindigkeitswerte im Wesentlichen dem Abluftgeschwindigkeits-Sollwert entsprechen.

7. Sicherheitswerkbank nach einem der vorhergehenden Ansprüche,
**dadurch gekennzeichnet,**
**dass** ein Positionssensor (18) zur Messung der Position der Frontscheibe (5) vorhanden ist.

8. Sicherheitswerkbank nach Anspruch 7,
**dadurch gekennzeichnet,**
**dass** in der Steuereinrichtung (15) vorherbestimmte Förderleistungswerte gespeichert sind, jeder Förderleistungswert jeweils einer Frontscheibenposition zugeordnet ist und die Steuereinrichtung (15) ausgebildet ist, die Förderleistung des wenigstens einen Gebläses (8) so zu steuern, dass die Förderleistung im Wesentlichen dem der jeweilig gemessenen Frontscheibenposition zugeordneten Förderleistungswert entspricht.

9. Sicherheitswerkbank nach Anspruch 7 oder 8,
**dadurch gekennzeichnet,**
**dass** in der Steuereinrichtung (15) eine Formel gespeichert ist, mittels derer für jede Frontscheibenposition ein entsprechender Förderleistungswert ermittelbar ist, und die Steuereinrichtung (15) ausgebildet ist, die Förderleistung des wenigstens einen Gebläses (8) so zu steuern, dass die Förderleistung im Wesentlichen dem für die jeweilig gemessene Frontscheibenposition ermittelten Förderleistungswert entspricht.

10. Sicherheitswerkbank nach einem der Ansprüche 5 bis 9,
**dadurch gekennzeichnet,**
**dass** die Regeleinrichtung (41) und/oder die Steuereinrichtung (15) mindestens ein Speichermedium (36) zur Speicherung der Sollwerte und/oder der Förderleistungswerte und/oder der Formel und mindestens einen Prozessor (40) zur Auswertung der ihr übermittelten Messwerte und zum Vergleich mit den gespeicherten Sollwerten und/oder Förderleistungswerten und/oder zur Berechnung der Förderleistungswerte umfasst.

11. Sicherheitswerkbank nach Anspruch 10,
**dadurch gekennzeichnet,**
**dass** sie ein Terminal und/oder eine Schnittstelle aufweist, mittels denen die Werte und/oder die Formel in das mindestens eine Speichermedium (36) eingebbar sind.

12. Sicherheitswerkbank gemäß einem der vorhergehenden Ansprüche,
**dadurch gekennzeichnet,**
**dass** die Frontscheibe (5) stufenweise, bevorzugt zwischen einer ersten (16) und einer zweiten Arbeitsposition (17), verstellbar ist.

13. Sicherheitswerkbank nach Anspruch 12,
**dadurch gekennzeichnet,**
**dass** die Höhe der Arbeitsöffnung (6) in der ersten Arbeitsposition (16) in einem Bereich von 10 cm bis 20 cm, bevorzugt 13 cm bis 17 cm, und in der zweiten Arbeitsposition (17) in einem Bereich von 30 cm bis 40 cm, bevorzugt 33 cm bis 37 cm, liegt.

14. Sicherheitswerkbank nach Anspruch 12 oder 13,
**dadurch gekennzeichnet,**
**dass** bei der ersten Arbeitsposition (16) der mittlere Schallpegel der Sicherheitswerkbank (1) während des Betriebes in einem Bereich von 50 dB bis 55 dB, bevorzugt 52 dB bis 53 dB, liegt.

15. Sicherheitswerkbank nach einem der Ansprüche 10 bis 14,
**dadurch gekennzeichnet,**
**dass** im Speichermedium (36) für unterschiedliche Betriebszustände der Sicherheitswerkbank (1) jeweils unterschiedliche Frontscheibenpositionen hinterlegt sind, zwischen denen die Frontscheibe (5) verfahrbar ist.

16. Sicherheitswerkbank nach einem der Ansprüche 7 bis 15,
**dadurch gekennzeichnet,**
**dass** sie ausgebildet ist, einen optischen und/oder akustischen Alarm (31) ausgeben, wenn die gemessene Höhe der Arbeitsöffnung (6) einen vorherbestimmten Maximalwert überschreitet.

17. Sicherheitswerkbank nach einem der vorhergehenden Ansprüche,
**dadurch gekennzeichnet,**
**dass** sie wenigstens eine Messvorrichtung (38) zur Bestimmung des Stromverbrauchs und/oder der Drehzahl des wenigstens einen Gebläses (8) aufweist.

18. Sicherheitswerkbank nach einem der vorhergehenden Ansprüche,
**dadurch gekennzeichnet,**
**dass** sie ein Sicherheitsüberwachungssystem (19) aufweist, welches mindestens ein Speichermedium (36) zum Speichern von Sollwertbereichen aufweist und welches, wenn es eine Abweichung
- der Lufteintrittsgeschwindigkeit oder
- der Abluftgeschwindigkeit oder
- des Stromverbrauchs des wenigstens einen Gebläses (8) oder
- der Drehzahl des wenigstens einen Gebläses (8)
von einem entsprechenden, gespeicherten Sollwertebereich feststellt, einen optischen und/oder akustischen Alarm (31) ausgibt.

19. Sicherheitswerkbank nach einem der vorhergehenden Ansprüche,
**dadurch gekennzeichnet,**
**dass** sie ein Sicherheitsüberwachungssystem (19) zur Überwachung der Funktionsfähigkeit wenigstens einer der folgenden Komponenten aufweist:
- Positionssensor (18)
- Strömungssensor (37)
- Messvorrichtung (38)
- Gebläse (8)
- Stromversorgung der Sicherheitswerkbank (1),
und das Sicherheitsüberwachungssystem (19) ausgebildet ist, bei einem Ausfall oder einem Defekt wenigstens einer der überwachten Komponenten einen optischen und/oder akustischen Alarm (31) und/oder mittels eines Displays (24) eine entsprechende Fehlermeldung auszugeben.
